# EUROPEAN PATENT SPECIFICATION

(11) **EP 0 549 478 B1**
(45) Date of publication and mention of the grant of the patent: **17.09.1997**
(21) Application number: 92403556.1
(22) Date of filing: 28.12.1992
(51) Int. Cl.: A23K 1/16, C08B 37/00, C07H 3/06

(54) **Method for preparing galacto-olisaccharides and feed for livestock containing the same**
Verfahren zur Herstellung von Galacto-Olichosacchariden und diese enthaltende Viefutter
Procédé de préparation de galacto-oligosaccharides et fourrages les contenant

(30) Priority: 27.12.1991 JP 359436/91
(43) Date of publication of application: 30.06.1993
(73) Proprietor: MATSUTANI CHEMICAL INDUSTRIES CO. LTD., Itami-shi, Hyogo-ken (JP)
(72) Inventor: Katta, Yasuo, Kako-gun, Hyogo-ken (JP); Ohkuma, Kazuhiro, Sanda-shi, Hyogo-ken (JP); Satouchi, Mitsuko, Takarazuka-shi, Hyogo-ken (JP); Takahashi, Reiji, Hyogo-ken (JP); Yamamoto, Takehiko, Izumi-shi, Osaka-fu (JP)
(74) Representative: Gillard, Marie-Louise

(56) References cited:
- EP-A- 0 133 547
- EP-A- 0 404 227
- EP-A- 0 435 657
- US-A- 4 083 733
- US-A- 4 873 229
- The Merck Index, 11th Edition

## Description

### BACKGROUND OF THE INVENTION

### 1. Field of the Invention

The present invention relates to a livestock feed containing galacto-oligosaccharides, and more particularly to a feed for livestock for alleviating diarrhea and discharge of soft feces which frequently occur in the weaning stage to ensure promoted growth, and to the method for preparing the galacto-oligosaccharides.

### 2. Description of the Prior Art

The improved productivity of livestock raising in Japan has become essential partly to meet increased demands for meat ensured by the Westernization of Japanese eating habits and partly to cope with intensified competition from imported meat supported by a prevailing trend for free trade. As one of the measures taken, it has been attempted to raise a large number of livestock at a higher density. This aggravates the raising environment to impose stress and causes diarrhea and discharge of soft feces, frequently leading to lower productivity in meat production. Especially, young livestock in the weaning stage tend to exhibit these symptoms partly due to an abrupt change in the environment. Improper growth at this stage adversely affects the rate of growth over the entire growth period entailing a lower feed efficiency and longer raising period.

To lessen these problems, it has been a practice to give antibiotics or antibacterial substances. However, strict restrictions are imposed on the kind of feeds to which such drugs can be added and the amount added, while recent concern is felt about the presence of drugs remaining in meat. Accordingly, it is desired to urgently develop feeds which obviate the need to use drugs. As to the mechanism of ameliorating diarrhea or discharge of soft feces, attention has been directed to increases in the amount of useful intestinal bacteria or to the inhibition of harmful bacteria. Based on the similar concept, live bacterial preparations of lactobacilli, Lactobacillus bifidus or the like are introduced into use, whereby they are unstable in efficiency and still remain to be improved.

Unexamined Japanese Patent Publication SHO 60-251896(1985) discloses a process for preparing galactosyl-lactose from lactose, i.e., a process for preparing oligosaccharides comprising glucose and galactose in the ratio of 1:2 or 1:3 by causing microorganisms of the genus Cryptococcus to act on lactose. Further, Unexamined Japanese Patent Publication SHO 63-109789(1988) discloses a process for preparing oligosaccharides comprising glucose and galactose in the ratio of 1:2 to 1:5 by causing beta-galactosidase to act on lactose. The oligosaccharides prepared by these processes contain up to 2% of glucose residues at the non-reducing ends.

Proposals are made of adding oligosaccharides to feeds for the relief of diarrhea and discharge of soft feces. Unexamined Japanese Patent Publication SHO 60-34134(1985) discloses a feed containing fructo-oligosaccharides, while Unexamined Japanese Patent Publication SHO 62-138147(1987) proposes addition of galactosyl lactose. Unexamined Japanese Patent Publication HEI 3-27255(1991) suggests usefulness of isomalto-oligosaccharides.

Nevertheless, these oligosaccharides have drawbacks in common. Since they are prepared by an enzymatic reaction, the reaction system is an aqueous system of relatively low concentration and produces effective oligosaccharides in a yield only up to about 55% on a dry solid basis. Moreover, the product contains large quantities of monosaccharides and disaccharides. The effective oligosaccharides have small molecular weight, are difficult to crystallize, and are usually distributed in syrup form. The product is difficult to uniformly mix with a feed when in the form of a syrup, and should preferably be a powder, whereas preparation of a powder product requires use of some additives. Otherwise, the powder obtained is highly hygroscopic, susceptible to caking and deliquescence and difficult to handle. Unexamined Japanese Patent Publication HEI 3-27255(1991) discloses an improved process wherein a silicate is used as a base agent for powdering isomalto-oligosaccharides, but even in this case, the content of effective oligosaccharides is only about 20 to about 25%. Thus the drawbacks of the prior-art oligosaccharides result in increases in cost, and it is desired to develop feeds which are less expensive and more effective.

### SUMMARY OF THE INVENTION

The main object of the invention is to provide a method for preparing galacto-oligosaccharides which consist essentially of glucose and galactose in the ratio of about 1:1, where at least 20% by weight of the galacto-oligosaccharides have a degree of polymerization of 3 to 11, said method comprising heating lactose in the presence of (a) an inorganic acid and (b) water in a small amount of up to 20% at a temperature of 120°C to 200°C for 5 to 20 seconds in an extruder.

Another object of the present invention is to provide a feed for livestock preferably for use in the growth period thereof where feed is free from the foregoing drawbacks of the prior art, is useful over the entire period of raising livestock and ameliorates diarrhea and discharge of soft feces which frequently occur in the suckling period for efficient growth of livestock.

The present invention provides a feed which contains galacto-oligosaccharides obtained by heating lactose in the presence of an inorganic acid in an extruder and which alleviates diarrhea and discharge of soft feces frequently occurring in the suckling period for efficient growth of livestock.

### DETAILED DESCRIPTION OF THE INVENTION

The term "galacto-oligosaccharides" as used hereinafter refers to oligosaccharides consisting essentially of glucose and galactose in the ratio of about 1:1 and obtained by heating lactose in the presence of an inorganic acid and water in a small amount of up to 20%, and galacto-oligosaccharides having a degree of polymerization of 3 to 11 will be referred to as "oligosaccharides." Remaining lactose and oligosaccharides are analyzed by high performance liquid chromatography, and amount of glucose residues at the non-reducing ends is analyzed by methylation analysis. The above amount of water refers to the water in the mixture of inorganic acid and water and water contained in the lactose, that is to the "total amount of water in the mixture of lactose and diluted mineral acid." The analytical values given are expressed in values of contents in dry solids, percentages and parts are by weight, and the values in parentheses are each an index obtained when the corresponding value of the control concerned is taken as 100.

The present invention relates to a feed for livestock which comprises as effective component galacto-oligosaccharides prepared by the method of appended claim 1 in an amount of 0.1 to 5 parts by weight per 100 parts by weight of feed. The feed is useful for livestock for alleviating diarrhea and discharge of soft feces during the entire raising period, more preferably for relieving such symptoms which occur especially frequently in the suckling period so as to readily ensure efficient growth of livestock.

Generally, the invention feed for livestock contains at least 20% by weight, and preferably 20 to 47% by weight of oligosaccharides, advantageously 30 to 47% by weight of oligosaccharides. On the other hand, the invention feed for livestock contains at least 10% by weight, and preferably about 10 to about 16% by weight of glucose residues at non-reducing ends thereof.

To ensure efficient growth of livestock, for example, young swine, to prevent mother swine from exhaustion due to nursing and to shorten mating interval of mother swine for efficient production, about 3-week-old sucklings are generally fed on a feed substituting for mother's milk and prepared for sucklings until they become about 2 months old, then fed on a feed for young swine until they become about 4 months old, and thereafter given a fattening feed. On the other hand, calves, especially milch cows for veal, are weaned 5 to 7 days after birth and fed on a feed for sucklings generally for 3 months after birth.

The present invention relates to a feed for such livestock, preferably to a feed to be given during a growth period following weaning until the fattening feed is given, and more preferably to a feed for use in the suckling period. The feed contains 0.1 to 5 parts of galacto-oligosaccharides per 100 parts of the feed, the oligosaccharides being prepared by heating lactose, which is a component of mother's milk, in the presence of a small amount of an inorganic acid, preferably hydrochloric acid in an extruder. The feed serves to alleviate diarrhea or discharge of soft feces during the entire raising period, preferably during the growth period, more preferably during the suckling period to ensure efficient growth of livestock.

The galacto-oligosaccharides for use in the present invention are prepared by adding about 500 ppm of an inorganic acid, preferably hydrochloric acid, to lactose and heat-treating the lactose at 100 to 200°C in an extruder. Unexamined Japanese Patent Publication HEI 3-197490(1991) discloses two processes for preparing galacto-oligosaccharides; one wherein lactose is heated in an anhydrous state in the presence of an inorganic acid, and the other comprising adding an inorganic acid to an aqueous solution of lactose, then spray-drying the mixture to obtain a dry powder and thereafter heating the powder. However, the product of galacto-oligosaccharides obtained by these processes contains only about 17% of oligosaccharides effective for ameliorating diarrhea and discharge of soft feces in livestock and promoting growth thereof as contemplated by the present invention and therefore fails to fully exhibit the contemplated effects. Moreover, the process wherein lactose is spray-dried before heating has the drawback of necessitating a spray dryer, including many steps and being complex and economically disadvantageous.

Accordingly, we have conducted research directing attention to heat treatment with use of an extruder as another production process and consequently obtained the novel finding that the content of effective oligosaccharides can be increased to about 50% by heat-treating lactose in the extruder with an inorganic acid, preferably hydrochloric acid, merely added to the lactose without necessitating spray drying. This finding has matured to the present invention which is economically outstanding.

Advantageously used as lactose in the present invention is any of commercial alpha-lactose, betalactose and spray dried lactose. Extruders, which are used for extruding materials under an increased pressure, are divided generally into those having a single rotative screw inserted in a cylinder, and those having two rotative screws in the same direction or different directions and inserted in cylinders arranged in the shape of the figure 8 in cross section. The screw or screws are generally demountable, and can be of various types with respect to the pitch, including reverse pitch type. Screws of varying pitches can be used in suitable combination. With some extruders, suitable screws are selectively usable in accordance with the properties of the material to be treated. Usually, the material is fed to the extruder at one end of the screw in rotation with the cylinder heated, and is treated as heated under an increased pressure, with the heat of friction between the screw and the material also being utilized.

Extruders are generally sized so that the screw is 30 to 340 mm in diameter, the ratio of the length of the screw to the diameter thereof being about 10:1 to about 45:1. They are heated with steam, or by electrical heating or induction heating. Actual operation of extruders requires material containers, material feeders, product cooler, product conveyor, product container, etc.

The extruder is heated to a temperature of 120 to 200°C for 5 to 20 seconds, preferably for about 10 seconds. To use the extruder as a reactor, it is required that the material and the product be smoothly moved through the extruder. Accordingly, the speed of rotation of the screw, which is closely related with the properties of the material lactose, heating temperature, reaction time and amount of acid added, must be determined optimally by measuring the content of oligosaccharides in the product. The speed is usually 120 to 400 r.p.m.

The most distinct feature of the heat treatment with use of the extruder is that lactose is reacted in a molten state in the presence of an inorganic acid. When heated by the conventional method, lactose is likely to remain a powder as in an untreated state, to be in a nearly crystalline state or to become a glasslike solid mass due to melting. Unlike such a conventional product, the lactose as heat-treated by the present process is in a brittle amorphous state since the lactose as melted under pressure is discharged from the extruder into the atmosphere. For this reason, galacto-oligosaccharides powder can be readily obtained when the treated product is lightly crushed as required.

The amount of hydrochloric acid to be added is about 2% based on lactose when the acid is in the form of an aqueous solution having a concentration of about 1%. Since the aqueous acid solution is added to powdered lactose, the lactose is heat-treated by stirring and aging of the mixture in a mixer to obtain a uniform mixture, predrying the resulting mixture at about 100 to about 120°C when required, and continuously feeding the mixture into the extruder as heated. The heat treatment is completed by rapidly cooling the product discharged from the outlet of the extruder.

Generally, the higher the reaction temperature, the higher the oligosaccharide content of the desired product, whereas an increased amount of colored substance will be formed at about 180°C and higher temperatures. Thus, it is not desirable to conduct the treatment at a very high temperature. More specifically, the reaction temperature is 120 to 200°C, preferably 130 to 180°C.

The galacto-oligosaccharides product obtained is added to a feed in an amount of 0.1 to 5 parts based on 100 parts of the resulting feed. If the amount is up to 0.1 part, the desired effect of the invention will not be fully obtained, whereas the presence of more than 5 parts of the product will not produce an improved effect and is therefore not practical. In this case, the galacto-oligosaccharides product can be added directly to the feed, or can be premixed with additives, such as vitamins, minerals, etc., to obtain a premix, which can then be added to the feed.

The feed of the present invention is prepared by adding the product to a usual livestock feed, preferably to a feed which is commercially available as a feed for growing livestock, or to the ingredients to be formulated into such a feed. For livestock in different stages of growth, feeds which are different in proportion of ingredients are commercially available. The galacto-oligosaccharides product is suitable for any of these feeds. Basically, there is no need to use additives, such as antibiotics, antibacterial substances or like drugs or live bacterial preparations, for alleviating diarrhea and discharge of soft feces, whereas conjoint use of some antibiotics appears effective as will be apparent from the examples given later, so that such an additive can be used in combination with the present galacto-oligosaccharides when so required.

The feed of the present invention is used for swine, calves and like livestock over the entire raising period, preferably in the growth period, exhibiting a remarkable effect to alleviate diarrhea and discharge of soft feces which frequently occur in the suckling period, reducing the feed conversion rate. It is also favorable in that the feed is suited to the taste of livestock, increases the amount of ingestion of feed and average weight gain per day and ensures growth with remarkably improved efficiency.

### Experimental Example 1

Commercial alpha-lactose (300*kg*) was placed into a ribbon mixer, 1% in concentration of 15 liters of hydrochloric acid solution was sprayed onto the lactose with pressurized air while rotating the mixer, and the lactose was subsequently passed through a disintegrator to prepare a uniform mixture, which was further treated in the ribbon mixer for 1 hour. The resulting mixture was continuously fed to a twinscrew extruder (Model TEX-32FSS-20AW-V, product of the Japan Steel Works, Ltd., diameter of screws 32 mm, for food use, selectively rotative in the same direction or different directions, motor output 7.5 kw, 400 r.p.m. max., screw length:diameter = 20:1, aluminum cast heater, water-cooling type, with vent holes) and heat-treated at 157, 160, 165, 167 or 170°C under the following conditions to obtain five kinds of galacto-oligosaccharides in a total amount of 250*kg*.
- Speed of rotation:: 150 r.p.m., rotation in same direction
- Inlet temperature:: room temperature (about 20°C)
- Reaction time :: 9 seconds

Table 1 shows analytical values of the galacto-oligosaccharides obtained.

**Table 1**

| Heating temp. (°C) | Remaining lactose | Oligosaccharides |
|---|---|---|
| 157 | 30.0 | 47.2 |
| 160 | 16.7 | 39.2 |
| 165 | 10.0 | 39.2 |
| 167 | 9.8 | 45.4 |
| 170 | 6.3 | 40.6 |

### Comparative Example 1

Commercial alpha-lactose (5*kg*) was placed into a ribbon mixer, 1% in concentration of 250*mℓ* of hydrochloric acid solution, was sprayed onto the lactose with pressurized air while rotating the mixer, and the lactose was subsequently passed through a disintegrator to prepare a uniform mixture, which was further treated in the ribbon mixer for 1 hour. The mixture was heated at 180°C, and during the period of 30 minutes after the start of heating until 180 minutes thereafter, 200*g* portions of the samples were collected at intervals of 30 minutes. Table 2 shows analytical values of the products.

**Table 2**

| Heating time (min) | Remaining lactose | Oligosaccharides |
|---|---|---|
| 30 | 75.8 | 15.0 |
| 60 | 68.8 | 18.9 |
| 90 | 68.7 | 17.7 |
| 120 | 67.0 | 15.3 |
| 150 | 70.5 | 15.9 |
| 180 | 63.0 | 17.3 |

The result of analysis shows that the oligosaccharide contents are less than 20% and are as low as about one-half of the values obtained in Experimental Example 1.

### Reference Example 1

About 260*kg* of galacto-oligosaccharides product was obtained by the same procedure as in Experimental Example 1 except that the mixture was heated at 168°C. The product had oligosaccharide content of 43.6%, consisted of glucose and galactose in the ratio of 1:0.99, contained a fraction of trisaccharide and higher oligosaccharides which was 960 in average molecular weight, and was 15.8% in the amount of glucose residues at the non-reducing ends. The galacto-oligosaccharides product will hereinafter be referred to as "sample 1."

### EXAMPLES

The present invention will be described below in greater detail with reference to examples.

### Example 1

In a swinery having a concrete floor, twenty-five 21-day-old young swine were divided into a control group and four example groups, 5 swine in each group, were raised for 7 weeks. The control group was given a feed of the composition shown in Table 3, while the example groups were given a mixture of the feed for the control group and sample 1 mixed therewith in an amount of 0.1, 0.5, 1.0 or 4.0 parts per 100 parts of the feed. The swine were raised in the same manner as is usual with swineries, with free access to the feed and water. The animals were checked for body weight at the start of raising and on completion of raising to determine the weight gain, and for the amount of feed ingested during the raising period to calculate the feed conversion rate. The animals were checked also for the occurrence of diarrhea and discharge of soft feces. If diarrhea or discharge of soft feces occurred in one swine a day, this case was counted as 1 point, and such points were summed up to obtain a diarrhea/soft feces score. Incidentally, diarrhea, if occurring, was immediately treated. Table 4 shows the results obtained.

**Table 3**

| Material | Amount (parts) |
|---|---|
| Wheat flour | 40 |
| Skimmed milk powder | 38 |
| Oil and fat | 3 |
| Fish powder | 5 |
| Glucose | 12 |
| Calcium carbonate | 0.3 |
| Calcium monohydrogen phosphate | 0.5 |
| Common salt | 0.2 |
| Vitamins | |
| Mineral mix | 1.0 |

**Table 4**

| Item | Control group | Example 1 | | | |
|---|---|---|---|---|---|
| Content of sample 1 (parts) | | 0.1 | 0.5 | 2.0 | 4.0 |
| Number | 5 | 5 | 5 | 5 | 5 |
| Initial average body weight(*kg*) | 5.7 | 5.7 | 5.7 | 5.7 | 5.7 |
| Final average body weight(*kg*) | 20.5 | 21.3 | 22.4 | 22.2 | 22.3 |
| Average weight gain per day(*g*) | 302 (100) | 318 (105) | 341 (113) | 337 (111) | 339 (112) |
| Average amount of feed ingested per day(*g*) | 456 (100) | 470 (103) | 487 (107) | 486 (107) | 484 (106) |
| Feed conversion rate | 1.51 (100) | 1.48 (98) | 1.43 (95) | 1.44 (95) | 1.43 (95) |
| Diarrhea/soft feces score | 48 (100) | 12 (25) | 4 (8) | 3 (6) | 5 (10) |

The results of raising shown in Table 4 reveal that the feed containing at least 0.1 part of sample 1 produced a remarkable effect to alleviate diarrhea or discharge of soft feces, further achieving improvements in weight gain and feed conversion rate. The contents of 0.5 to 4 parts are comparable in effect. In view of economy, therefore, the preferred content is about 0.5 part.

### Example 2

In a usual swinery, 23-day-old young swine were divided into three groups, i.e., a control group, example group and reference group, 34 swine in each group, and raised for 38 days. The raising period was divided into the former period of 10 days and the latter period of 28 days. Commercial formula feeds having the respective compositions given in Table 5 for raising sucklings were fed to the control group in the former and latter periods. The example group was fed on a mixture of 99.5 parts of each feed for the control group and 0.5 part of sample 1. The reference group was fed on a mixture of 99.5 parts of each feed for the control group and 0.5 part of commercial fructo-oligosaccharides as adapted to contain about 90% of effective component by fractionation.

**Table 5**

| Composition(%) of formula feeds for growing sucklings | | |
|---|---|---|
| Component | For former period | For latter period |
| Crude protein | At least 21.0 | At least 18.0 |
| Crude fat | 3.5 | 4.5 |
| Crude fiber | 2.0 | 8.5 |
| Crude ash | 8.0 | 8.0 |
| Calcium | 0.60 | 0.60 |
| Phosphorous | 0.45 | 0.45 |
| Soluble protein | 19.5 | 15.5 |
| Total amount of soluble nutrients | 85.0 | 80.0 |

The swine were fed, maintained and checked in the same manner as in Example 1. The commercial feed used in the former period contained 40*g* value/ton of colistin sulfate and 88*g* value/ton of tyrosine phosphate, and the one used in the latter period contained 88*g* value/ton of tyrosine phosphate.

The results of raising obtained in the former period are listed in Table 6, and those achieved in the latter period in Table 7.

**Table 6**

| Item | Control group | Reference group | Example 2 |
|---|---|---|---|
| Number of swine | 34 | 34 | 34 |
| Initial average body weight(*kg*) | 6.5 | 6.4 | 6.4 |
| Final average body weight(*kg*) | 8.8 | 8.8 | 8.8 |
| Average weight gain per day(*g*) | 232 (100) | 241 (104) | 243 (105) |
| Average amount of feed ingested per day(*g*) | 328 (100) | 310 (95) | 319 (97) |
| Feed conversion rate | 1.41 (100) | 1.29 (91) | 1.31(93) |
| Diarrhea/soft feces score | 9 (100) | 4 (44) | 1 (11) |

**Table 7**

| Item | Control group | Reference group | Example 2 |
|---|---|---|---|
| Number of swine | 34 | 34 | 34 |
| Initial average body weight(*kg*) | 8.8 | 8.8 | 8.8 |
| Final average body weight(*kg*) | 19.9 | 20.2 | 20.2 |
| Average weight gain per day(*g*) | 394 (100) | 408 (104) | 408 (104) |
| Average amount of feed ingested per day(*g*) | 700 (100) | 719 (103) | 738 (105) |
| Feed conversion rate | 1.78 (100) | 1.76 (99) | 1.81(102) |
| Diarrhea/soft feces score | 1 (100) | 0 ( 0) | 0 ( 0) |

Although the feed used contained antibiotics in an upper limit amount regulated under the law, diarrhea occurred during the former period of weaning, whereas this symptom remarkably ameliorated in the case of the example group. Although this effect was observed also with the commercial product of fructo-oligosaccharides, sample 1 was found more effective. No significant effect was observed in the weaning latter period. This appears attributable to the function of the antibiotics which acted favorably in one way or another in this stage. Even in such a situation, improvements were achieved in weight gain and feed conversion rate in the case of the example group.

### Example 3

Twenty-five male Holsteins, 5 to 7 days old, were divided into five groups, i.e., four example groups and one control group, 5 calves in each group, and were raised for 1 month. The control group was fed on a commercial milk substitute for growing sucklings, while the example groups were given a mixture of each 99.9, 99.5, 98.0, 95.0 parts of the feed for the control group and 0.1, 0.5, 2.0 or 5.0 parts of sample 1 respectively. During the raising period, the calves were checked for the occurrence of diarrhea, weight gain and feed conversion rate. Table 8 shows the results.

**Table 8**

| Item | Control group | Example 3 | | | |
|---|---|---|---|---|---|
| Content of sample 1(parts) | - | 0.1 | 0.5 | 2.0 | 5.0 |
| Initial average body weight(*kg*) | 41 | 40 | 40 | 41 | 40 |
| Final average body weight(*kg*) | 57.5 | 57.0 | 58.0 | 58.5 | 57.5 |
| Average weight gain per day(*g*) | 589 (100) | 607 (103) | 643 (109) | 625 (106) | 625 (106) |
| Feed conversion rate | 0.63 (100) | 0.60 (95) | 0.57 (90) | 0.56 (89) | 0.57 (90) |
| Dierrhea/soft feces | Frequent | Infrequent | Seldom | Seldom | Seldom |

When sucklings were fed on the feed containing sample 1, occurrence of diarrhea or discharge of soft feces was significantly lessened with improvements achieved in weight gain and feed conversion rate.

### Example 4

A sample 1 (1*kg*) was dissolved in 1 liter of water, and the solution was fed to a column packed with a strongly basic ion exchange resin (packed with 100 liters of DOWEX XFS-43279.00, Na type product of Dow Chemical, Japan) at 65°C, SV 0.5 After saccharide component was eluted from the column, 5-liter fractions were collected as Fr. 1, 2, 3 and 4. This procedure was repeated five times, and then fraction Fr. 4 was collected, concentrated, freeze-dried to obtain powders, i.e., about 0.5*kg* of low-molecular-weight fraction of galacto-oligosaccharides. This fraction will be referred to as "sample 2." Sample 2 had a disaccharide content of 1.6% and tri-to polysaccharide content of 95.9%, 90.2% of which were oligosaccharides. These oligosaccharides were almost all trisaccharides and were 560 in average molecular weight.

Fifteen 37-day-old young swine were divided into three groups, i.e. a control group and two example groups, 5 swine in each group, and were raised for 28 days in the same manner as in Example 1. The control group was fed on a commercial feed for growing sucklings, while the example groups were given a mixture of the feed for the control group and 0.5 part of sample 1 and sample 2. Table 9 shows the results.

**Table 9**

| Item | Control group | Example 4 | |
|---|---|---|---|
| | - | Sample 1 | Sample 2 |
| Average weight gain per day (*g*) | 369 (100) | 409 (111) | 428 (116) |
| Average amount of feed ingested per day (*g*) | 624 (100) | 657 (105) | 673 (108) |
| Feed conversion rate | 1.69 (100) | 1.61 (95) | 1.57 (93) |
| Diarrhea/soft feces score | 11 (100) | 1 ( 9) | 0 ( 0) |

The example groups were superior to the control group in the result attained. Especially, sample 2 consisting primarily of oligosaccharides exhibited a better result. This result indicates that the high-molecular-weight fraction is slightly inferior in the effect of the invention.

### Example 5

Twelve 23-day-old young swine were divided into a control group and an example group, 6 swine in each group, and were raised for 6 weeks. The control group was fed on the feed of the composition of Table 3, while the example group was given a mixture of 99.5 parts of the feed for the control group and 0.5 part of sample 1. The swine were raised in the same manner as in Example 1, with the results listed in Table 10.

**Table 10**

| Item | Control group | Example 5 |
|---|---|---|
| Number of swine | 6 | 6 |
| Initial average body weight (*kg*) | 6.3 | 6.3 |
| Final average body weight (*kg*) | 23.2 | 24.3 |
| Average weight gain per day (*g*) | 403 (100) | 426 (106) |
| Average amount of feed ingested per day (*g*) | 589 (100) | 590 (100) |
| Feed conversion rate | 1.46 (100) | 1.38 (95) |
| Diarrhea/soft feces score | 6 (100) | 1 (17) |

Galacto-oligosaccharides were found effective also by this example.

## Claims

1. A method for preparing galacto-oligosaccharides which consists essentially of glucose and galactose in the ratio of about 1:1, where at least 20 % by weight of the galacto-oligosaccharides have a degree of polymerization of 3 to 11, said method comprising heating lactose in the presence of (a) an inorganic acid and (b) water in a small amount of up to 20 % at a temperature of 120°C to 200°C for 5 to 20 seconds in an extruder.

2. A method of claim 1, wherein 20 to 47 % by weight of the galacto-oligosaccharides have a degree of polymerization of 3 to 11.

3. A method of claim 1 or 2, wherein 30 to 47 % by weight of the galacto-oligosaccharides have a degree of polymerization of 3 to 11.

4. A method of any one of claims 1 to 3, wherein the galacto-oligosaccharides contain 10 to 16 % by weight of glucose residues at the non reducing ends thereof.

5. A feed for livestock, characterized in that the feed contains galacto-oligosaccharides which are prepared by the method of any one of claims 1 to 4.

6. A feed for livestock of claim 5 which contains 0.1 to 5 parts by weight of the galacto-oligosaccharides per 100 parts by weight of the feed.

7. A feed for livestock of claim 5 or 6 which is a feed for sucklings of livestock.

8. A feed for livestock of any one of claims 5 to 7 which is a feed for swine and calf sucklings.

## Patentansprüche

1. Verfahren zur Herstellung von Galacto-Oligosacchariden, die im wesentlichen aus Glucose und Galactose im Verhältnis von etwa 1:1 bestehen, wobei wenigstens 20 Gew.% der Galacto-Oligosaccharide einen Polymerisationsgrad von 3 bis 11 haben, wobei das Verfahren Erhitzen von Lactose für 5 bis 20 Sekunden bei einer Temperatur von 120°C bis 200°C in einem Extruder in Gegenwart von (a) einer anorganischen Säure und (b) Wasser in einer kleinen Menge von bis zu 20% umfaßt.

2. Verfahren nach Anspruch 1, in dem 20 bis 47 Gew.% der Galacto-Oligosaccharide einen Polymerisationsgrad von 3 bis 11 aufweisen.

3. Verfahren nach Anspruch 1 oder 2, in dem 30 bis 47 Gew.% der Galacto-Oligosaccharide einen Polymerisationsgrad von 3 bis 11 aufweisen.

4. Verfahren nach einem der Ansprüche 1 bis 3, in dem die Galacto-Oligosaccharide 10 bis 16 Gew.% Glucosereste an ihren nicht-reduzierenden Enden enthalten.

5. Viehfutter, dadurch gekennzeichnet, daß das Futter Galacto-Oligosaccharide enthält, die durch das Verfahren nach einem der Ansprüche 1 bis 4 hergestellt wurden.

6. Viehfutter nach Anspruch 5, das 0,1 bis 5 Gewichtsteile der Galacto-Oligosaccharide je 100 Gewichtsteilen Futter enthält.

7. Viehfutter nach Anspruch 5 oder 6, das Futter für nicht entwöhntes Vieh ist.

8. Viehfutter nach einem der Ansprüche 5 bis 7, das Futter für nicht entwöhnte Schweine und Kälber ist.

## Revendications

1. Procédé de préparation de galacto-oligosaccharides constitutés essentiellement de glucose et de galactose dans le rapport d'environ 1:1, au moins 20 % en poids des galacto-oligosaccharides ayant un degré de polymérisation de 3 à 11, ledit procédé comprenant le chauffage de galactose en présence (a) d'un acide inorganique et (b) d'eau en une petite quantité atteignant au maximum 20 % à une température comprise entre 120°C et 200°C pendant 5 à 20 s dans une extrudeuse.

2. Procédé selon la revendication 1, dans lequel 20 à 47 % en poids des oligosaccharides ont un degré de polymérisation de 3 à 11.

3. Procédé selon la revendication 1 ou 2, dans lequel 30 à 47 % en poids des galacto-oligosaccharides ont un degré de polymérisation de 3 à 11.

4. Procédé selon l'une quelconque des revendications 1 à 3, dans lequel les galacto-oligosaccharides contiennent 10 à 16 % en poids de résidus glucose à leurs extrémités non réductrices.

5. Aliment pour bétail, caractérisé en ce que l'aliment contient des galacto-oligosaccharides préparés par le procédé selon l'une quelconque des revendications 1 à 4.

6. Aliment pour bétail selon la revendication 5, lequel contient 0,1 à 5 parties en poids de galacto-oligosaccharides pour 100 parties en poids d'aliment.

7. Aliment pour bétail selon la revendication 5 ou 6, lequel est un aliment pour bétail au stade animaux de lait.

8. Aliment pour bétail selon l'une quelconque des revendications 5 à 7, lequel est un aliment pour cochons de lait et veaux de lait.
